Europäisches Patentamt

European Patent Office

Office européen des brevets

(11) Publication number: **0 420 819 A1**

## EUROPEAN PATENT APPLICATION

(21) Application number: **90810733.7**

(22) Date of filing: **24.09.90**

(51) Int. Cl.5: **C12N 15/82**, C12N 15/29, A01H 5/00

(30) Priority: **25.09.89 DE 3931969**

(43) Date of publication of application:
**03.04.91 Bulletin 91/14**

(84) Designated Contracting States:
**AT BE CH DE DK ES FR GB GR IT LI LU NL SE**

(71) Applicant: **Max-Planck-Gesellschaft zur Förderung der Wissenschaften e.V.**
**Bunsenstrasse 10**
**W-3400 Göttingen(DE)**

(72) Inventor: **Logemann, Jürgen**
**Am Welschbach 7**
**W-5042 Erfstadt(DE)**
Inventor: **Schell, Jeff**
**Carl-von-Linné-Weg 10**
**W-5000 Köln 30(DE)**
Inventor: **Siebertz, Barbara**
**Kirchenhof 2**
**W-5000 Köln 41(DE)**

(74) Representative: **Smolders, Walter et al**
**c/o Sandoz Technology Ltd., Lichtstrasse 35**
**CH-4002 Basel(CH)**

(54) Improvements in or relating to organic compounds.

(57) DNA sequences are described, which enable genes operably linked to them to be specifically expressed in anthers. By using anther-specific promoters, it is i.a. possible to obtain male sterility.

EP 0 420 819 A1

## IMPROVEMENTS IN OR RELATING TO ORGANIC COMPOUNDS

The invention relates to a DNA sequence, an anther-specific promoter and the use thereof.

The creation of cultivated plants with male sterility has been of economic interest for a long time, since this prevents self-pollination which proliferates among many plant species and hinders breeders when producing new cultivars.

Male sterility is of particular interest in the production of hybrid seeds. Many mono- and dicotytedoneous cultivated plants, such as cereals, vegetables and ornamentals, are sold as hybrid seeds. Production of these hybrid seeds is simplified considerably by the male sterility of one of the two parental lines. Some cultivated plants which have male sterility achieved by selection are already known. However, it is difficult to obtain such plants.

A gene with a DNA sequence called "wun1 gene", which is isolated from Solanum tuberosum, is known from literature references such as The Plant Cell, Vol. 1, 1989, p. 151-158; Proc. Natl. Acad.Sci.USA, Vol. 85, p. 1136-1140, Fbr.1988, as well as DE-OS 38 37 752. It is known that the wun1 gene leads to the expression of gene products in the case of wounding or a pathogenic infection. It could be shown that in the case of the "wun1 gene" a promoter region of 1022 bp length ($+$179bp wun1 5′ untranslated region) maintains its wound-inducible activity even when the actual structural gene region is replaced by another structural gene (e.g. CAT, GUS). In this way, wound-inducible wun1 promoter activity could be detected in leaf, stem and root of transgenic tobacco plants.

In contrast to DE-OS 38 37 752, which is concerned with a wound-stimulated DNA sequence and its use, the present invention relates to anther-specifically expressed DNA sequences and their use. DNA sequences were established, which enable structural genes to be expressed specifically in anthers.

It has been found that the wun1 gene known from the references to literature already mentioned can not only be used as wound and/or infection-induced DNA sequence, but if the promoter of the wun1 gene is shortened, an anther-specifically expressed DNA sequence may be attained. The range necessary to distinguish this feature extends over 1201 base pairs (bp) (1022 (bp) of the wun1 promoter $+$ 179 bp of the 5′untranslated region). If the 1201 base pair length is reduced, e.g. by removal of 451 bp of the wun1 promoter at the 5′end, this shortened promoter is only still active in the anthers. This can be proved with a reporter gene, i.e. a gene producing a selectable or visibly screenable change of the phenotype in the transformed cell or plant, when attached to the shortened promoter. The change in the phenotype, e.g. colouration cannot be found in any part of the plant except for the pollen and stomium of anthers.

In this way, if GUS is employed as reporter gene and for example 451 bp ( -571wun1-GUS) are removed at the 5′-end of the wun1 promoter, and this truncated promoter is tested e.g. in transgenic tobacco plants, there is no longer any activity in wounded or unwounded leaves, stems and roots.

The truncated promoter shows however surprisingly a constitutive activity in the anthers.

For convenience, 1201 bp of the wun1 gene consisting of the promoter region of 1022 bp and the 5′ untranslated region of 179 bp attached at the 3′end of the promoter region is herein designated -1022 wun1. Fragments thereof shortened at the 5′ end by x (e.g. 451) bp are designated (-1022 $+$x)-wun1 (e.g. -571wun1). Vectors having such promoter (e.g. -571wun1) attached at the 5′end of the US gene are designated (-1022 $+$ x) wun1-GUS (e.g. -571 wun1-GUS).

The same expression behaviour observed with -571 wun1-GUS is also found with other wun1 promoter fragments, e.g. with -86wun1-GUS.

The invention therefore provides a DNA sequence of a DNA fraction of the 5′ upstream region of the wun1 gene, DNA sequences homologous thereto and parts thereof, said DNA sequences having constitutive anther specific promoter activity.

The term DNA sequence homologous to a DNA sequence of a constitutive anther specific promoter of the invention refers to a DNA sequence of a constitutive anther specific promoter of the invention wherein a number of nucleotides have been deleted and/or added but is still capable of hybridization to a nucleotide sequence complementary to a DNA sequence of a constitutive anther specific promoter of the invention under appropriate hybridization conditions. For the purpose of the invention appropriate hybridization conditions conveniently include an incubation for 16 hours at 42°C, in a buffer system comprising 5 x standard saline citrate (SSC), 0.5 % sodium dodecylsulphate (SDS), 5 x Denhardt's solution, 50 % formamide and 100 $\mu$g/ml carrier DNA (hereinafter the buffer system), followed by washing 3 times with a buffer comprising 1 x SSC and 0.1 % SDS at 65°C for approximately one hour each time.

Preferred hybridization conditions for the purpose of the invention involve incubation in the buffer system for 16 hours at 49°C and washing 3 times with a buffer comprising 0.1 x SSC and 0.1 % SDS at 55°C for approximately one hour each time. Most preferred hybridization conditions for the purpose of the invention involve incubation in the buffer system for 16 hours at 55° C and washing 3 times with a buffer comprising 0.1 x SSC and 0.1 % SDS at 65° C for approximately one hour each time.

Suitable examples of promoter elements according to the invention having constitutive anther specific promoter activity are the base pair sequences

a) from -571 to +179,
b) from -571 to + 1,
c) from -86 to +179,
d) from -86 to + 1,
e) from -571 to -111 3′ linked with the -86 to +179 sequence and
f) from -571 to -111 3′ linked with the -86 to +1 sequence, of the wun1-gene, DNA sequences homologous thereto, and parts thereof.

Other DNA sequences having constitutive anther specific promoter activity may be derived from the -1022wun1 promoter by eliminating base pairs thereof and screening such shortened promoter for competitive anther specific promoter activity in a manner known per se, e.g. employing a reporter gene such as GUS, analogous to the procedure described herein.

The promoters of the invention are useful, in that they allow the selective expression of gene products in the anthers. For that purpose, a promoter of the invention is operably linked to a gene of which the selective expression of the gene product in the anthers is desired, e.g. to inhibit or influence cell development in the anthers or for ornamental purposes.

The invention accordingly provides transformation vectors comprising a constitutive anther specific promoter according to the invention operably linked to a gene.

Examples of genes of which anther specific expression may be useful include genes causing development aberrations including male sterility, e.g. the rolC gene, or genes encoding gene products which inactivate the plant cell physiology, e.g. the thionin gene, genes encoding gene products capable of degrading DNA, e.g. the gene encoding the synthesis of the Eco RI endonuclease or by inactivation of genes essential for anther development, e.g. the wun1 antisense gene. Anther specific expression of such genes will result in male sterile plants.

For the production of F1 hybrid seed the availability of a restorer system is necessary if the F1 hybrid crop involved is grown for its fruits or its seeds. For use in the present invention such restorer system can be made by using the anti-sense gene of the gene that was used to induce male sterility in the transgenic plant. Methods to achieve anti-sense inhibition are known to a person skilled in the art and could include e.g. the anti-sense rol C gene if the Δwun1-rolC construct was used to create male sterility; a similar strategy can be used for the thionine gene; in cases of the endonuclease gene induced male sterility not only the anti-sense strategy could be used, but also the insertion of the corresponding methylase gene creating a restorer for the endonuclease induced male sterility.

Combination of a genetically engineered male sterility in the mother line with a male line containing the appropriate restorer gene will result in a fertile F1 hybrid product capable of yielding normal amounts of fruits or seeds.

Male sterility brought about by gene technology has the advantage that by incorporating a definite gene into the genetic code of a plant, the remaining properties of the plant do not change. This applies especially to cultivated plants which genetically can be manipulated relatively well, e.g. to dicotyledoneous plants which are suited for tissue culture and transformable, but also to other cultivated plants.

It is of course possible to anther-specifically express genes which do not induce male sterility but have other properties of interest to breeders, e.g. gene products affecting the anthocyanine biosynthesis route to give coloured anthers in flowers having petals that have white or different colours. The latter possibility is of great interest for breeders of ornamentals.

The invention also provides male sterile plants or plant material comprising a constitutive anther specific promoter according to the invention.

Such plants or plant material may be obtained in a manner known per se employing conventional transformation techniques, and where desired, followed by conventional breeding of thus obtained transgenic plants.

The following demonstrates that deletion of the wun1 promoter, which is known from the abovementioned references to literature, leads to anther-specific expression.

In the figures are illustrated:

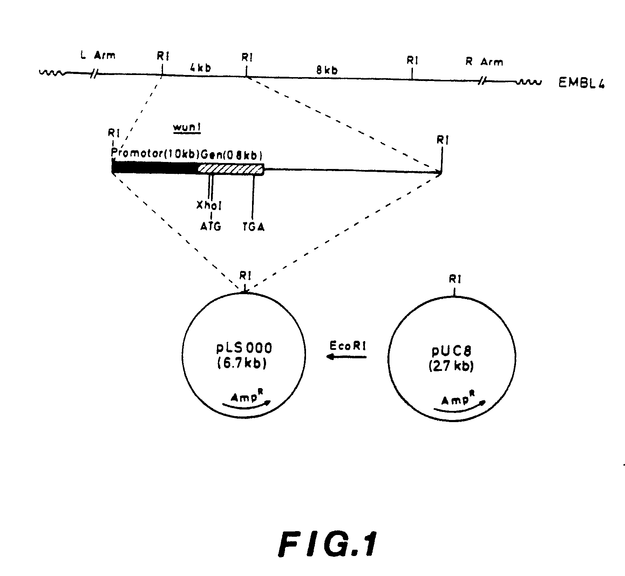

Fig. 1: Arrangement of wun1 in the genomic clone wun1-85. 1.0 Kb of the wun1 promoter, 0.8 Kb of the wun1 gene coding region and 2.0 Kb of the 3′ end are present on the 4 Kb EcoRI fragment of the genomic clone. This 4 Kb fragment was cloned into the EcoRI cutting site of pUC8 (pLS000).

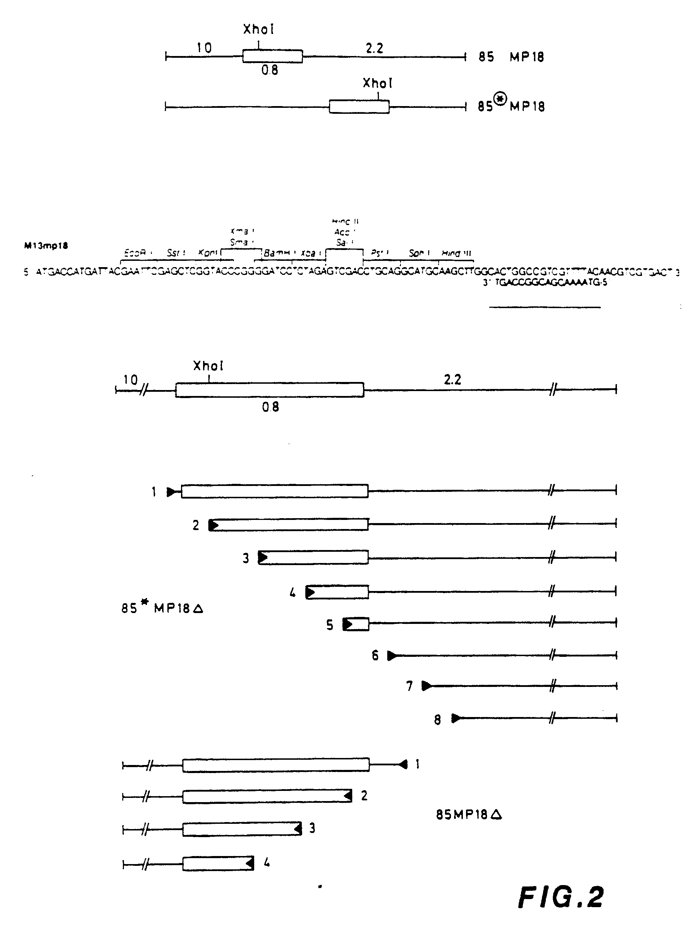

Fig. 2: Deletion analysis of the wun1-genomic clone wun1-85. On the basis of the asymmetrical XhoI cutting site in the genomic clone wun1-85, the 4 Kb fragment could be detected in both orientations in M13mp18 (85MP18; 85*MP18). DNA fragments of various sizes were obtained by successive digestion with exonucleaseIII.

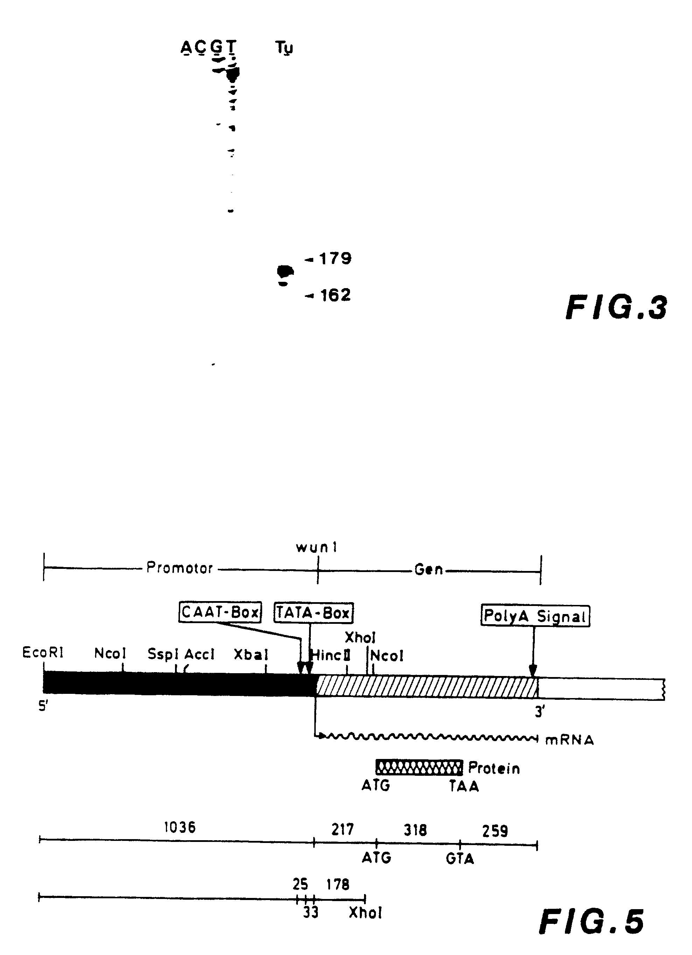

Fig. 3: Determination of the wun1 transcription start point. Wun1-mRNA from wounded potato tubers was hybridised against the 1.2 Kb EcoRI/XhoI Fragment of pLS000. This leads to a DNA-RNA hybrid which was protected against the single-strand-specific S1 nuclease. As shown on a denaturing polyacrylamide-gel (lane TU) the length of the nuclease-protected DNA fragment is 162-179 bp. The actual start of transcription is thus 162-179 Bp 5′ distant from the XhoI cutting site (A,C,G,T = sequence diagram to determine the size of the DNA fragment).

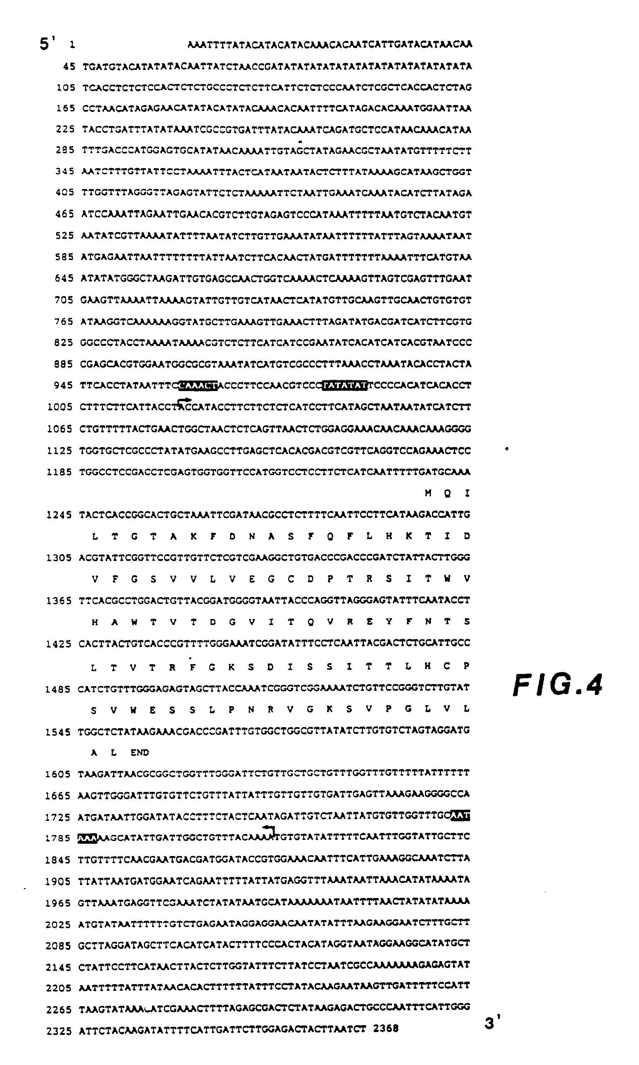

Fig. 4: Nucleotide sequence of wun1 and flanking regions from wun1-85. CAAT-Box, TATA-Box and PolyA-signal are shown on black backgrounds. The start and stop of transcription are marked by arrows.

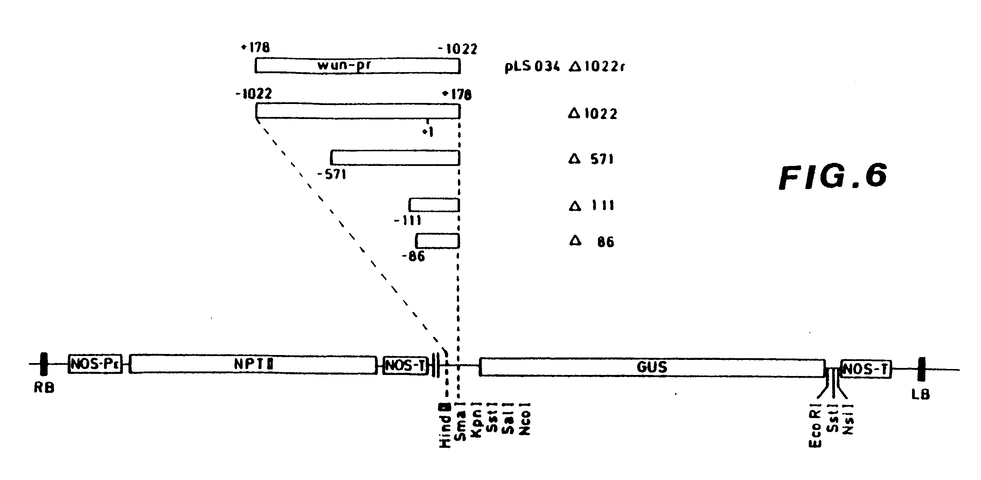

Fig. 5: Arrangement and position of important regions in the wun1 gene. The wun1 promoter is marked in black, the wun1 gene is marked in lines. Important recognition sequences are framed; the mRNA is indicated by a wavy line, and the protein-coding region by crosses. The size of the individual regions is given in (bp).

Fig. 6: Construction of wun1-GUS 5′ deletions for expression analysis in stable tobacco transformants. Between the left and the right T-DNA border sequence of the vector pPR69, a NPT II gene lies under the control of the nopalin synthase promoter (nos). This is necessary for selection for kanamycin. The GUS gene bears a nos-terminator sequence and is regulated by the various 5′deletion fragments of the wun1 promoter. The end positions of the respective 5′deletion fragments are given together with the respective construction.

R = cloning of the promoter in reversed orientation.

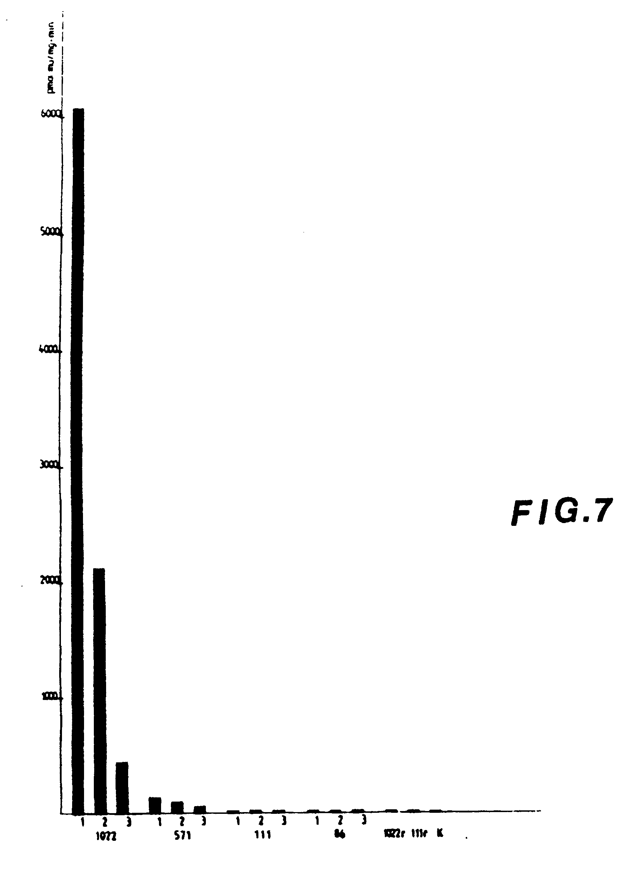

Fig. 7: Tobacco leaves of transgenic plants from the greenhouse were analysed for their GUS activity, depending on the various 5′promoter elements. The unit pmol MU/mg protein/min on the abscissa indicates enzyme activity. The ordinates respectively show 3 out of 6-12 various tested plants. These plants reflect the highest, the average and the lowest activity of the respective tested plants, following wounding.

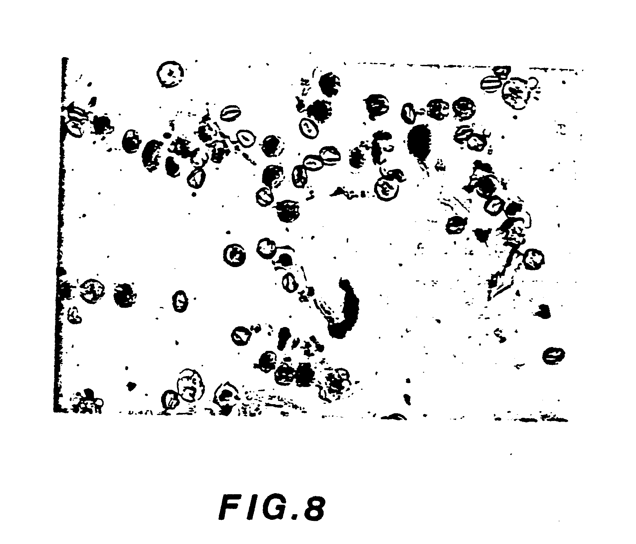

Fig. 8: Germinating pollen of the construction pLS034-571. The blue colouring in the pollen and in the pollen tube indicates the location of enzyme activity.

MATERIAL AND METHODS

Reference is made to the following literature:

(1) Ansorge, W., De Mayer, L. (1980). Thermally stabilized very thin (0.02-0.3 mm) polyacrylamide gels for electrophoresis. J. Chromatogr., 202: 45-33.

(2) Ansorge, W., Barker, R. (1984). System for DNA sequencing with resolution of up to 600 base pairs. J. Biochem.. Biophys. Meth., 9:33-47.

(3) Bedbrook, J. (1981). A plant nuclear DNA preparation procedure. P.M.B. Newsletter II, 24.

(4) Benoist, C., O'Hare, K., Breathnach, R., Chambon, P. (1980). The ovalbumin gene sequence of putative control regions. Nucl. Acids Res., 8:127-142.

(5) Benton, W.D. and Davis, R.in. (1977). Screening Lambda gt recombinant clones by hybridization to single plaques in situ . Science, 196:180.

(6) Berk, A. and Sharp, P.A. (1987). Spliced early mRNAS of SV40. Proc. Natl. Acad. Sci. USA, 75:1274-1278.

(7) Bevan, M. (1984). Binary Agrobacterium vectors for plant transformation. Nucl. Acids Res. 12: 8711-8721.

(8) Ebert, P.R., HA, S.B., An, G. (1987). Identification of an essential upstream element in the nopalin synthase promoter by stable and transient assays. Proc.Natl.Acad.Sci USA 84: 5745-5749.

(9) Fickett, F.W. (1982). Recognition of protein coding regions in DNA sequences. Nucl. Acids Res., 10:5303-5318.

(10) Frischauf, A.H., Lehrach, H., Poustka, A. and Hurray, N. (1983). Lambda replacement vectors carrying polylinker sequences. J. Mol. Biol., 170:827-842.

(11) Henikoff, S. (1984). Unidirectional digestion with exonuclease III creates targeted breakpoints for DNA sequencing. Gene, 28:351-359.

(12) Hoekema, A., Hirsch, P., Hooykaas, P., Schilperoort, R. (1983). A binary plant vector strategy based on separation or vir- and T-region of A.tumefaciens . Nature 303: 179-180.

(13) Hohn, B. and Murray, K. (1977). Packaging recombinant DNA molecules into bacteriophage particles in vitro. Proc. Natl. Acad. Sci. USA, 74: 3259-3263.

(14) Jefferson, R.A. (1987). Assaying chimeric genes in plants: the GUS gene fusion system. Plant Mol.Biol.Rep. 5: 387-405.

(15) Joshi, C.P. (1987). An inspection of the domain between putative TATA box and translation start side in 79 plant genes. Nucl. Acids Res. 15: 6643-6653.

(16) Lehrach, H., Diamond, D., Wozney, J.M. and Boedtker, H. (1977). RNA molecular weight determinations by gele electrophoresis under denaturing conditions, a critical re-examination. Biochemistry, 16:4743.

(17) Lipphardt, S. (1988). Dissertation an der Universität zu Köln.

(18) Logemann, J., Schell, J. and Willmitzer, L. (1987). Improved method for the isolation of RNA from plant tissues. Anal. Biochem., 163; 16-20.

(19) Maniatis, T., Fritsch, E.F., Sambrook, J. (1982). Molecular cloning: a laboratory manual. Cold Spring Harbour Laboratory, Cold Spring Harbour, New York.

(20) Messing, J., Geraghty, D., Hu, N.T., Kridl, J. and Rubenstein, I. (1977). Plant Gene Structure. In: Kosuge, F., Meredith, C.P., Hollaender, A. (eds.). Genetic engineering of plants. Plenum Press, New York: 211-227.

(21) Murashige, T., Skoog, F. (1962). A rapid method for rapid growth and bioassays with tobacco tissue cultures. Physiol. Plant., 15:473-497.

(22) Murray, M.G., Thompson, W.F. (1980). Rapid isolation of high molecular weight plant DNA. Nucl.Acids.Res. 8: 4321-4325.

(23) Reiss, B., Sprengel, R., Will, H., Schaller, H. (1984). A new and sensitive method for qualitative and quantitative analysis of neomycinphosphotransferase in crude cell extracts. Gene 30:217-223.

(24) Sanger, F., Nicklen, S., Coulson, A.R. (1977). DNA sequencing with chain-terminating inhibitors. Proc. Natl. Acad. Sci. USA, 74:5463-5467.

(25) Southern, E.M. (1975). Detection of specific sequences among DNA fragments separated by gel electrophoresis. J. Mol. Biol., 98:503-517.

(26) Van Houte, E., Joos, H., Maes, M., Warren, G., Van Montagu, M., Schell, J. (1983). Intergenic transfer and exchange recombination of restriction fragments cloned in pBR322: a novel strategy for reversed genetics of Ti-plasmids of Agrobacterium tumefaciens . EMBO J., 2:411-418.

(27) Vieira, J., Messing, J. (1982). The pUC plasmid, an M13mp7-derived system for insertion mutagenesis and sequencing with synthetic universal primers. Gene, 19:259-268.

(28) Wassenegger, M. (1988). Dissertation an der Universität zu Köln.

(29) Willmitzer, L., Schmalenbach, W. and Schell, J. (1981). Transcription of T-DNA in octopine and nopaline crown gall tumours is inhibited by low concentration of alfa-amanitin Nucl. Acids Res., 9:19.

(30) Yanisch-Perron, C., Vieira, J., Messing, J. (1985). Improved M13 phage cloning vectors and host strains: nucleotide sequencing of the M13mp18 and puc19 vectors. Gene 33: 103-119.

(31) Zambrisky, P., Joos, J., Genetello, C., Leemans, J., Van Montagu, M., Schell, J. (1983). Ti-Plasmid vector for the introduction of DNA into plant cells without alteration of their normal capacity. EMBO J., 1:147-152.

## MATERIAL

### Media

The media used for the culture of bacteria were taken from data given by Maniatis et al. (1982).

Plant media:

The media employed are derived from the media (MS) given by Murashige and Skoog (1962).

3MS : MS + 3 % saccharose

3MSC : MS + 3 % saccharose, 500 ug/ml claforan

MSC15 : MS + 2 % saccharose, 500 ug/ml claforan, 100 ug/ml kanamycin sulphate

MSC16 : MS + 0.5 ug/ml BAP + 0.1 ug/ml NAA + 100 ug/ml kanamycin sulphate + 500 ug/ml claforan.

For solid medium, an additional 8 g/l bacto-agar was added.

### Strains and vectors

E. coli strains:

BMH 71-18: Δ (lac-proAB), thi, supE; F'(lacI$^q$, ZdeltaM15, proA$^+$ B$^+$) (Messing et al. 1977)

C 600 : also known as CR34 (Maniatis et al., 1982)

Agrobacteria strains: LBA 4404: (Hoekema et al., 1983)

Plasmids: pUC8 (Vieira and Messing, 1982) pPR69 (a derivative of bin 19, Bevan 1984)

Phages: EMBL4 (Frischauf et al., 1983) M13mp18 (Yanisch-Perron et al., 1985) M13mp19 ( Yanisch-Perron et al., 1985)

Plants: Solanum tuberosum AM 80/5793 (haploid)

Nicotiana tabacum Wisconsin 38 (W38)

### METHODS

If not otherwise stated, all standard molecular-biological methods, e.g. restriction analysis, plasmid isolation, mini preparation of plasmid-DNA, transformation of bacteria etc., were carried out as described by Maniatis et al., (1982).

## RNA isolation

Isolation of RNA from various organs of the potato and of tobacco were carried out as described by Logemann et al., (1987).

## "Northern-Blot" Analysis

The RNA was separated by electrophoresis on a 1.5% formaldehyde-agarose-gel (Lehrach et al., (1977)). As described by Willmitzer et al., (1982), the RNA was subsequently transferred onto nitrocellulose, fixed and hybridized on $^{32}$P radioactively-labelled cDNA, washed and exposed.

## DNA isolation

Nuclear DNA was recovered by the method of Bedbrook (1981) from potato leaves and used for cloning in lambda phages EMBL4. DNA from transformed tissue was purified by combined lyses with Triton X 100, SDS, and proteinase K (Wassenegger, 1988).

## "Southern Blot" Analysis

DNA was separated by electrophoresis on 0.8 to 1.2% agarose gels, transferred onto nitrocellulose and fixed (Southern, 1975), also hybridized and washed as described by Willmitzer et al., (1981).

## Establishment and research of a genomic bank

### Isolation of genomic DNA

The genomic potato DNA used was leaf DNA of the haploid species AM 80/5793, isolated by the method of Bedbrook, 1981. This DNA was digested totally with EcoRI.

### Isolation of the phages DNA

EMBL4 DNA was dissected with EcoRI into three fragments, whereby the two vector arms could be separated from the middle fragment by means of gel electrophoresis separation with subsequent fragment isolation. In addition, commercially available, purified EMBL4 arms were also used (Amersham).

### Ligation and packaging

Following ligation of the EMBL4 arms with the ECORI-digested genomic DNA, the ligated, high molecular weight DNA is packaged in vitro into phage heads (Hohn and Murray, 1977; Hohn, 1979). The packaging material stems from a "Lambda in vitro packaging kit" from the company Amersham. The genomic bank was plated out at a concentration of 25000 plaques per plate (25x25 cm).

### Plaque hybridization

The discovery of cDNA homologous lambda clones was made by means of plaque hybridization according to Benton and Davies (1977) using radioactively labelled cDNA. Positive hybridizing plaques were isolated and tested again.

### DNA preparation of recombinant phages

20 ml of a bacteria culture, lysed with C600, are mixed with chloroform, in order to obtain a bacteria-free supernatant after subsequent centrifugation. Sedimentation of the phages from the supernatant is effected by centrifugation for 4 hours at 10000 rpm. The phage sediment is taken up in 500 $\mu$l phage buffer (10 mM tris-HCl pH 8.0, 10 mM MgCl$_2$), and treated with DNase and RNase. After extraction of the DNA by means of phenolization several times, the phage-DNA is EtOH-precipitated, washed with 70 % EtOH and taken up in TE.

### Production of deletion mutants following exonuclease-III treatment (Henikoff et al., 1984)

The fragment to be examined was cloned in M13mp19 in both orientations. Since exonuclease III digests 5$^{'}$ projecting ends, but spares 3$^{'}$ projecting ends, 20 ug of the DNA to be analysed was digested with two restriction enzymes which produced a 5$^{'}$ and a 3$^{'}$ end on one side of the cloned fragment. Since the 5$^{'}$ end was turned towards the fragment, exonuclease digestion into the fragment could take place. By successive stopping of this reaction, fragments which were 200 bp smaller could be isolated, and the projecting ends thereof were transformed into ligatable smooth ends by means of subsequent S1-treatment. Finally, transformation of this DNA into BMH 7118 cells took place, with subsequent single-stranded DNA isolation.

Sequencing

Sequencing of single-stranded DNA was carried out by the chain-terminating method of Sanger et al., (1977). Separation of the reaction products was performed on 6% and 8% sequencing gels (Ansorge and de Mäyer, 1980; Ansorge and Barker, 1984).

S1 analysis

Determination of the starting point of transcription was effected according to Berk and Sharp (1987). For this purpose, the 1.2 kb fragment was isolated from the plasmid pLS000 with EcoRI and Xhol, and dephosphorylated by treatment with phosphatase. Then, radioactive labelling of the 5′ OH ends took place through the combination of polynucleotide kinase and y- $^{32}$P-ATP. After denaturing of the DNA fragments, they were hybridized with 50 ug of whole RNA (hybridization buffer: 80% formamide, 0.4 M NaCl, 40 mM PIPES pH 6.4 and 1 mM EDTA. This condition favours RNA-DNA hybrids compared with DNA-DNA hybrids (Casey and Davidson, 1977). The temperature at the start of hybridization starts at 80° C and is lowered over night to 40° C. Subsequent S1-nuclease digestion (120 U/ml) removes unpaired strands. It was expected that the radioactively labelled Xhol cutting site found in the 5′ untranslated region of the wun1 gene would be protected by its homology to the mRNA. Finally, the S1 protected DNA strand is separated by electrophoresis on a sequencing gel, whereby the sequence of a known DNA fragment is used for length comparison.

DNA transfer in agrobacteria

**Transformation**

The DNA cloned in E. coli was transferred to A. tumefaciens LBA4404 (Hoekema et al., 1983) by the method described by Van Haute et al., (1983), by conjugation.

**DNA analysis**

Identification of DNA transfer to the Agrobacterium was effected by isolation of Agrobacterium DNA according to the method depicted by Ebert et al. (1987). Restriction cleavage of the DNA, the transfer to nitrocellulose and the hybridization on the corresponding radioactive sample provided evidence of the successful DNA transfer into the Agrobacterium.

Transformation of tobacco

**Culture of Agrobacterium**

The Agrobacterium strains containing the appropriate LBA4404 vector required for infection were grown in selective antibiotic medium (Zambrisky et al., 1983), sedimented by centrifugation and washed in YEB medium without antibiotics. After further sedimentation and absorption in 3MS medium, the bacteria could be used for infection.

**Infection of leaf sections**

For infection of leaf sections, sterile leaves of tobacco species SNN and W38 were used. Leaf pieces of about 1 cm$^2$ size, immersed into the Agrobacterium suspension as described above, with subsequent transfer onto 3MS medium. After incubation for 2 days at 16 hours light and 25° C to 27° C, the pieces were transferred onto MSC16 for callus and shoot induction. Shoots appearing after 4-6 weeks were cut off and incubated on MSC15 medium.

Analysis of the transformed plants

**Isolation of genomic DNA**

The DNA from the plants is isolated by a varied method of Murray and Thompson (1980), cut with restriction enzymes and separated on a gel. After transfer of this DNA onto nitrocellulose, it is hybridized with a radioactively labelled sample, which indicates the presence of this specific DNA in the genome of the plant under examination.

**Detection of GUS activity**

Plants regenerated from transformation are examined using the method of Jefferson (1987) for the degree of activity of the enzyme β-glucuronidase (GUS). An additional histochemical test (Jefferson, 1987) indicates localisation in the tissue. For this purpose, thin sections of the plants are incubated with the substrate X-Gluc, 5-bromo-4-chloro-3-indolyl glucuronide. A blue precipitate forms at the site of enzyme activity.

## RESULTS

### Establishment of a genomic bank from a haploid potato

The small number of wun1 genes in the genome of the haploid potato cultivar AM80/5793 led to the construction of a genomic bank consisting of genomic DNA which is digested with EcoRI.

10 ug of EcoRI cleaved DNA from AM80/5793 were ligated with EcoRI cleaved EMBL4 arms and plated on a C600 bacterial lawn. Ca. 500,000 plaques were obtained, which taken statistically, should represent the genome of the potato.

### Identification of wun1 homologous EMBL4 clones

After the transfer of these plaques onto nitrocellulose, by using plaque hybridization techniques on radioactively labelled wun1-cDNA, two genomic clones (wun1-22 and wun1-85) could be identified and purified.

Isolation of recombinant EMBL4 DNA from wun1-22, wun1-85, as well as their restriction mapping and hybridization on radioactive wun1- cDNA, gave the following data:

The wun1-cDNA equivalent fragment in wun1-85 has a size of 4 Kb. Thus, it corresponds exactly to the fragment size of EcoRI digested DNA from the haploid potato, which hybridizes with wun1-cDNA. Based on the asymmetrical XhoI cutting site in the 5′ region of the cDNA clone, and the use of radioactive cDNA samples which only cover the 5′ region of the cDNA clone, the orientation of the gene in the 4 Kb fragment could be determined. Accordingly, 5′ of the wun1 gene there is a promoter region of ca. 1 Kb size, while at 3′ from the gene there is a non-homologous part of ca. 2 Kb (fig. 5). The EcoRI fragment of 8 Kb size additionally contained in wun1-85 could not be referred to for closer analysis of the wun1 gene. Based on the digestion of the potato DNA with EcoRI, it is probable that during the subsequent ligation, two fragments not belonging together were brought into the EMBL4 vector, that is, neither can they enter a functional relationship with one another.

### Sequence analysis of the genomic clone wun1-85

In the following, the genomic analysis of the wun1 coding gene was carried out. Since in the restriction behaviour, no differences cound be established between wun1-85 and wun1-22, the 4 Kb fragment of wun1-85 was ligated into Eco RI cleaved pUC8 for further analysis (fig. 1). In order to sequence the wun1 promoter and also the wunl gene, further re-cloning of the 4 Kb fragment into the Eco RI cutting site of M13mp18 took place. Determination of its orientation was carried out using control digestion at the asymmetrical XhoI cutting site. The clones 85*mp18 and 85mp18 represent both orientations of the fragment (fig. 62. Cutting of these plasmids with SphI and XbaI enabled successive digestion, using exonucleaselII, of the 3′ end of the wun1 gene in the case of clone 85mp18 and the 5′ end of the wunl gene in the case of clone 85*mp18 to take place.

Deletion clones resulting therefrom, with varying fragment sizes, were employed for sequencing. In all, it was possible to sequence the entire wun1 gene bidirectionally, and additionally to analyse ca. 400 bp of the 3′ end unidirectionally (fig. 4).

### Determination of the transcription starting point

In order to identify the exact start of transcription of the wun1 gene, the method of S1 nuclease mapping was used. The principle of this method is based on the fact that as a result of hybridization of single-stranded DNA from the 5′ region of the wun1 gene with wun1-mRNA, only the regions which because of their homology may form a double strand are protected from degradation of the single-strand-specific nuclease S1. The size of the protected DNA fragment can be determined on a sequencing gel and thus the start of transcript ion can be followed back.

Assisted by the cutting sites Xho I and Eco RI, a 1.2 Kb fragment was isolated from pLS000, and it contains the promoter and parts of the 5′ untranslated region of the wun1 gene. This fragment was radioactively labelled with $^{32}$P with polynucleotide kinase at the 5′ end of the Xho I site, and hybridized on 50 ug of total RNA from wounded potato tubers. After S1 treatment of the hybrid, the size of the protected fragment was determined on a sequencing gel; it varies between 162-179 bp (fig. 3). When starting from the longest fragment, the start of transcription begins 179 bp upstream from the XhoI site, i.e. with the sequence AC**CAT**AC. This sequence conforms in the central region (**CAT**) with the consensus sequence, determined by Joshi et al., 1987, for the start of transcription CT**CATC**A. Further information is provided from the position of the start of transcription (figs. 4, 5):

1.) In position -33, viewed from the start of transcription, a TATA box C**TATATAT**T is found, which conforms well with the consensus sequence TCAC**TATATAT**AG determined by Joshi et al., 1987.

2.) The CAAT box described by Benoist et al., 1980, in the region between -60 and -80 can be found in the wun1 promoter at position -58 (CAAACT).

3.) The 5' untranslated region of the wun1 gene extends over 217 bp and is therefore comparatively large.

4.) The wun1-mRNA coding gene thus encloses 794 bp, which corresponds very exactly with the size determination of wun1-mRNA based on "Northern-Blot" analysis.

5.) 97 bp of the 5' untranslated region in the wun1 gene are missing in the cDNA clone wun1-25A2.

6.) Apart from the open reading frames already determined in the cDNA clone, no further branches can be found in the 5' untranslated region of the genomic clone.

7.) The wun1 gene has no introns at its disposal.

## Establishment of the derivatives of the vector pPR69

The promoter deletion fragments produced by means of exonuclease III with the end points -571 bp, -111 bp and -86 bp, as well as the promoter up to the region -1022 bp in the 5' region, were recloned into vector pPR6g (Fig. 6). For preferential recloning, the deletion fragments beyond the XhoI cutting site were cut out of M13mp18. To produce the control, which bears the vector in reversed orientation, the 5' overlapping ends of the cutting sites were filled by the Klenow fragment of DNA-polymerase I and cloned into the vector. Absorption of the deletion fragments in the vector was tested by hybridization on a radioactively labelled wun1 promoter sample and by restriction analysis.

## Stable transformation and analysis of wun1-GUS in tobacco

Various wun1 promoter deletions in transgenic plants were tested for their organ specifity and their wound inducibility. For this purpose, the plasmids pLS034-1022, -571, -111, -89, -1022R were transformed into the agrobacterial strain LBA4404. These agrobacteria were then used for transformation of the tobacco cultivar Wisconsin 38 (W38) by means of the leaf section infection method. Under kanamycin selection, the callus produced on the plant were regenerated and were examined on Southern Blot for correct integration of the pLS034-DNA into the plant genome.

The functional analysis of the transgenic tobacco plants was extended to the detection of GUS activity in greenhouse plants.

Independent transformants of different constructions were analysed for their GUS activity in various tissues. Non-transformed tobacco plants served as a control (K).

Comparison with the GUS activity in tobacco leaves showed little activity for plants of the construction pLS034-86, -111 and pLS034-1022R, which was at about the level of activity of control plants. Compared with these, the GUS activity of pLS034-571 is ca. 4 times higher, while the activity of the whole promoter is 13 to 370 times greater than that of the shortest construction (pLS034-86), see fig. 7.

The activity of the enzyme in various tissues of the plant was analysed by means of histochemical in-situ analysis. For this, thin sections of the tissue were incubated over night at 37°C with the substrate of the enzyme, 5-bromo-4-chloro-3-indolyl glucuronide (X-Gluc). A blue precipitate forms at the site of enzyme activity. This analysis indicated that in plants of constructions pLS034-86, -111, -571 and -1022R, no activity can be detected in leaves, stem or roots.

Plants carrying the full wun1 promoter in their construction show activity in all the said tissues.

Another picture was seen during the analysis of flowers. In cross-sections of flowers of the various constructions, there was the following distribution of enzyme activity: Plants of construction pLS034-1022 showed GUS activity in the stomium of the anthers and in the pollen grain. 30% of the pollen grain showed blue colouring after incubation with X-Gluc. Anthers of pLS034-571 and -86 showed the same pattern of activity, but with less intensity (Fig. 8). In control plants, as with plants of construction pLS034-111 and pLS034-1022R, no blue colouring could be detected in the stomium, and colouring of the pollen grain was reduced to 0.75%. Comparison of the measured GUS activity of anthers from the various constructions showed 2-10 times stronger activity of pLS034-1022 than in pLS034-571. The average enzyme activity of the full promoter in anthers reached 1276 pmol MU/mg/min. Anthers of the construction pLS034-571 showed 2-times higher activity than anthers of pLS034-86. The average enzyme activity is 217 pmol MU/mg/min, resp. 97 pmol MU/mg/min.

As is known, the 35S promoter of cauliflower mosaic virus is active in anthers. Its activity is 500 pmol MU/mg/min. Since the 35S promoter of the CaMV is a strong promoter which is active in plants, the deletion fragments of the wun1 promoter extend into this activity range throughout.

**Claims**

1. DNA sequence of the 5′ upstream region of the wun1 gene, DNA sequences homologous thereto and parts thereof, said DNA sequences having constitutive anther specific promoter activity.

2. DNA sequence according to Claim 1, characterised in that the 5′ upstream region of the wun1 gene is derived from the 1201 base pairs long DNA fraction consisting of the 1022 base pairs of the wun1 promoter region and the 5′ wun1 untranslated region of 179 base pairs attached to the 3′ end of the promoter region.

3. DNA sequence according to Claim 2, with a bp range of from -571 of the wun1 promoter to +179 of the 5′ untranslated region of the wun1 gene, DNA sequences homologous thereto and parts thereof.

4. DNA sequence according to Claim 3, with a bp range of

    a) from -571 of the wun1 promoter to +179 of the 5′ untranslated region of the wun1 gene, and DNA sequences homologous thereto, or

    b) from -571 of the wun1 promoter to +1 of the 5′ untranslated region, and DNA sequences homologous thereto, or

    c) from -86 of the wun1 promoter to +179 of the 5′ untranslated region, and DNA sequences homologous thereto, or

    d) from -86 of the wun1 promoter to +1 of the 5′ untranslated region, and DNA sequences homologous thereto, or

    e) from -571 to -111 of the wun1 promoter 3′ linked with the DNA sequence of from -86 of the wun1 promoter to +179 of the 5′ untranslated region, and DNA sequences homologous thereto, or

    f) from -571 to -111 of the wun1 promoter 3′ linked with the DNA sequence of from -86 of the wun1 promoter to +1 of the untranslated region, and DNA sequences homologous thereto.

5. The use of a DNA sequence according to Claims 1 to 4 to obtain pollen/anther specific expression.

6. The use according to Claim 5 for the preparation of plants having increased ornamental value or of male sterile plants.

7. Transformation vectors comprising a constitutive anther specific promoter of Claims 1 to 4 operably linked to a gene.

8. Plants or plant material containing a constitutive anther-specific promoter according to Claims 1 to 4.

# FIG.1

FIG.2

FIG.3

FIG.5

EP 0 420 819 A1

```
5'  1                          AAATTTTATACATACATACAAACACAATCATTGATACATAACAA
    45 TGATGTACATATATACAATTATCTAACCGATATATATATATATATATATATATATATA
   105 TCACCTCTCTCCACTCTCTGCCCTCTCTTCATTCTCTCCCAATCTCGCTCACCACTCTAG
   165 CCTAACATAGAGAACATATACATATACAAACACAATTTTCATAGACACAAATGGAATTAA
   225 TACCTGATTTATATAAATCGCCGTGATTTATACAAATCAGATGCTCCATAACAAACATAA
   285 TTTGACCCATGGAGTGCATATAACAAAATTGTAGCTATAGAACGCTAATATGTTTTTCTT
   345 AATCTTTGTTATTCCTAAAATTTACTCATAATAATACTCTTTATAAAAGCATAAGCTGGT
   405 TTGGTTTAGGGTTAGAGTATTCTCTAAAAATTCTAATTGAAATCAAATACATCTTATAGA
   465 ATCCAAATTAGAATTGAACACGTCTTGTAGAGTCCCATAAATTTTTAATGTCTACAATGT
   525 AATATCGTTAAAATATTTTAATATCTTGTTGAAATATAATTTTTTATTTAGTAAAATAAT
   585 ATGAGAATTAATTTTTTTTATTAATCTTCACAACTATGATTTTTTTAAAATTTCATGTAA
   645 ATATATGGGCTAAGATTGTGAGCCAACTGGTCAAAACTCAAAAGTTAGTCGAGTTTGAAT
   705 GAAGTTAAAATTAAAAGTATTGTTGTCATAACTCATATGTTGCAAGTTGCAACTGTGTGT
   765 ATAAGGTCAAAAAAGGTATGCTTGAAAGTTGAAACTTTAGATATGACGATCATCTTCGTG
   825 GGCCCTACCTAAAATAAAACGTCTCTTCATCATCCGAATATCACATCATCACGTAATCCC
   885 CGAGCACGTGGAATGGCGCGTAAATATCATGTCGCCCTTTAAACCTAAATACACCTACTA
   945 TTCACCTATAATTTCCAAACTACCCTTCCAACGTCCCTATATATTCCCCACATCACACCT
  1005 CTTTCTTCATTACCTACCATACCTTCTTCTCTCATCCTTCATAGCTAATAATATCATCTT
  1065 CTGTTTTTACTGAACTGGCTAACTCTCAGTTAACTCTGGAGGAAACAACAAACAAAGGGG
  1125 TGGTGCTCGCCCTATATGAAGCCTTGAGCTCACACGACGTCGTTCAGGTCCAGAAACTCC
  1185 TGGCCTCCGACCTCGAGTGGTGGTTCCATGGTCCTCCTTCTCATCAATTTTTGATGCAAA
                                                              M  Q  I
  1245 TACTCACCGGCACTGCTAAAATTCGATAACGCCTCTTTTCAATTCCTTCATAAGACCATTG
        L  T  G  T  A  K  F  D  N  A  S  F  Q  F  L  H  K  T  I  D
  1305 ACGTATTCGGTTCCGTTGTTCTCGTCGAAGGCTGTGACCCGACCCGATCTATTACTTGGG
        V  F  G  S  V  V  L  V  E  G  C  D  P  T  R  S  I  T  W  V
  1365 TTCACGCCTGGACTGTTACGGATGGGGTAATTACCCAGGTTAGGGAGTATTTCAATACCT
        H  A  W  T  V  T  D  G  V  I  T  Q  V  R  E  Y  F  N  T  S
  1425 CACTTACTGTCACCCGTTTTGGGAAATCGGATATTTCCTCAATTACGACTCTGCATTGCC
        L  T  V  T  R  F  G  K  S  D  I  S  S  I  T  T  L  H  C  P
  1485 CATCTGTTTGGGAGAGTAGCTTACCAAATCGGGTCGGAAAATCTGTTCCGGGTCTTGTAT
        S  V  W  E  S  S  L  P  N  R  V  G  K  S  V  P  G  L  V  L
  1545 TGGCTCTATAAGAAACGACCCGATTTGTGGCTGGCGTTATATCTTGTGTCTAGTAGGATG
        A  L  END
  1605 TAAGATTAACGCGGCTGGTTTGGGATTCTGTTGCTGCTGTTTGGTTTGTTTTTATTTTTT
  1665 AAGTTGGGATTTGTGTTCTGTTTATTATTTGTTGTTGTGATTGAGTTAAAGAAGGGGCCA
  1725 ATGATAATTGGATATACCTTTCTACTCAATAGATTGTCTAATTATGTGTTGGTTTGCAAT
  1785 AAAAAGCATATTGATTGGCTGTTTACAAATGTGTATATTTTTCAATTTGGTATTGCTTC
  1845 TTGTTTTCAACGAATGACGATGGATACCGTGGAAACAATTTCATTGAAAGGCAAATCTTA
  1905 TTATTAATGATGGAATCAGAATTTTTATTATGAGGTTTAAATAATTAAACATATAAAATA
  1965 GTTAAATGAGGTTCGAAATCTATATAATGCATAAAAAAATAATTTTAACTATATATAAA
  2025 ATGTATAATTTTTTGTCTGAGAATAGGAGGAACAATATATTTAAGAAGGAATCTTTGCTT
  2085 GCTTAGGATAGCTTCACATCATACTTTTCCCACTACATAGGTAATAGGAAGGCATATGCT
  2145 CTATTCCTTCATAACTTACTCTTGGTATTTCTTATCCTAATCGCCAAAAAAAGAGAGTAT
  2205 AATTTTTATTTATAACACACTTTTTTATTTCCTATACAAGAATAAGTTGATTTTTCCATT
  2265 TAAGTATAAACATCGAAACTTTTTAGAGCGACTCTATAAGAGACTGCCCAATTTCATTGGG
  2325 ATTCTACAAGATATTTTCATTGATTCTTGGAGACTACTTAATCT 2368          3'
```

*FIG.4*

14

*FIG.6*

pLS 034  Δ1022r

Δ 1022

Δ 571

Δ 111

Δ 86

+178  wun-pr  -1022

+178

-1022  +178

+1

-571

-111

-86

NOS-T

Nsi I
Sst II
Eco RI

GUS

Nco I
Sal II
Sst I
Kpn I
Sma I
Hind III

NOS-T

NPT II

NOS-Pr

RB

LB

*FIG.7*

FIG.8

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | THE PLANT CELL, vol. 1, January 1989, pages 151-158, American Society of Plant Physiologists; J. LOGEMANN et al.: "5' Upstream sequences from the wun1 gene are responsible for gene activation by wounding in transgenic plants" <br> * Whole document * | 1,2,7,8 | C 12 N 15/82 <br> C 12 N 15/29 <br> A 01 H 5/00 |
| O,X | JOURNAL OF CELLULAR BIOCHEMISTRY, suppl. 13D, 1989, page 306, abstract no. M 331, Alan R. Liss, Inc., New York, US; J. LOGEMANN et al.: "5' Upstream sequences from the wun1 gene are responsible for gene-activation by wounding and tissue specific expression in transgenic plants" <br> * Abstract no. M 331 * | 3,4,5,7,8 | |
| O,Y | IDEM | 6 | |
| O,Y | JOURNAL OF CELLULAR BIOCHEMISTRY, suppl. 13D, 1989, page 292, abstract no. M 257, Alan R. Liss, Inc., New York, US; A.J. VAN TUNEN et al.: "Regulation of chalcone flavanone isomerase (CHI) gene-expression in Petunia Hybrida: the use of alternative promoters in corolla, anthers and pollen" <br> * Abstract no. M 257 * | 6 | TECHNICAL FIELDS SEARCHED (Int. Cl.5) <br><br> C 12 N <br> A 01 H |
| P,X | THE PLANT CELL, vol. 1, October 1989, pages 961-968, American Society of Plant Physiologists; B. SIEBERTZ et al.: "cis-Analysis of the wound-inducible promoter wun1 in transgenic tobacco plants and histochemical localization of its expression" <br> * Page 965, left-hand column, paragraph 2 * | 1-5,7,8 | |

−/−

The present search report has been drawn up for all claims

| Place of search | Date of completion of search | Examiner |
|---|---|---|
| The Hague | 17 December 90 | MADDOX A.D. |

European
Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | WO-A-9 005 187  (MAX-PLANCK-GESELLSCHAFT)<br>* Page 42, paragraph 2 *<br>– – – | 1,2,5,7,8 | |
| A | EP-A-0 329 308  (PALADIN HYBRIDS)<br>* Page 12, lines 1-20; example 6 *<br>– – – – – | 6 | |

TECHNICAL FIELDS
SEARCHED (Int. Cl.5)

The present search report has been drawn up for all claims

| Place of search | Date of completion of search | Examiner |
|---|---|---|
| The Hague | 17 December 90 | MADDOX A.D. |

CATEGORY OF CITED DOCUMENTS

X : particularly relevant if taken alone
Y : particularly relevant if combined with another
  document of the same catagory
A : technological background
O : non-written disclosure
P : intermediate document
T : theory or principle underlying the invention

E : earlier patent document, but published on, or after
  the filing date
D : document cited in the application
L : document cited for other reasons

&: member of the same patent family, corresponding
  document